# DEMANDE DE BREVET EUROPEEN

(11) **EP 2 277 452 A1**
(43) Date de publication de la demande: **26.01.2011**
(21) Numéro de dépôt: 09166265.0
(22) Date de dépôt: 23.07.2009
(51) Int. Cl.: A61B 8/13, G01S 15/66, G01S 15/87, G01S 15/89

(54) **Dispositif de guidage d'ultrasons**

(71) Demandeur: Siemens Schweiz AG, 8047 Zürich (CH)
(72) Inventeur: Cloutot, Laurent, 8956, Killwangen (CH); Broennimann, Thomas, 4704, Wolfisberg (CH)
(74) Mandataire: Fischer, Michael

(57) **Abrégé**

Dispositif de guidage d'ultrasons apte à être piloté pour visualiser et localiser spatialement une zone limitée (Z), la dite zone étant une zone in-vivo d'un sujet, comprenant :
- une pluralité de parties émettrices/réceptrices d'ultrasons, chacune étant reliée à au moins une sonde à ultrasons (S, S1, S2, S3,...),
- les parties émettrices/réceptrices d'ultrasons forment un réseau de canaux (C1, C2, C3, ...) de guidage d'ultrasons vers le sujet, lesdits canaux étant distinctement espacés sur une surface d'un plan (T) contre lequel au moins une partie du sujet est superficiellement appuyée,
- un module de contrôle (CTRL) est relié à une unité de commutation (SW) qui active le guidage d'ultrasons au travers d'au moins un des canaux dits activés et désactive le guidage d'ultrasons au travers des autres canaux dits désactivés.

## Description

La présente invention concerne un dispositif de guidage d'ultrasons selon le préambule de la revendication 1.

Actuellement, un objectif majeur réside dans la visualisation de parties internes telles qu'in-vivo chez un sujet de type humain ou animal ou un mannequin dit aussi « fantôme ». En particulier, cet objectif est crucial dès lors qu'une intervention telle qu'une irradiation locale radiothérapeutique ou technologiquement équivalente en dépend afin de pouvoir localement irradier une zone limitée incluant des cellules à détruire tout en préservant de l'irradiation de tissus et cellules saines avoisinés aux cellules malsaines.

Actuellement par exemple, lors d'une séance de radiothérapie sur des cellules cancéreuses telle qu'une tumeur ou un carcinome, un dispositif annexe à l'installation de radiothérapie permet de prendre des prises de vue de type radiographique afin de visualiser et localiser ladite tumeur in-vivo dans le référentiel de l'installation de radiothérapie, ceci aux fins de contrôler que le faisceau irradiant concentre bien son énergie sur la tumeur. Ce procédé à base d'irradiation nuisible (rayons X) n'est toutefois pas sans danger pour un patient déjà affaibli par un cancer et ne permet pas une visualisation ainsi qu'une localisation d'une cible à intervalles fréquents et donc a fortiori en temps réel lors de séances d'irradiation. De tels procédés sont aussi volumineux, coûteux et nécessitent de multiples interventions humaines pour leur positionnement, leur fonctionnement et leur maintenance. Il en ressort que le pilotage d'une cible de faisceau irradiant sur une zone in-vivo lors d'un diagnostic, d'un planning ou d'une thérapie se révèle encore trop statique et donc imprécis au cas où la zone in-vivo à cibler se déplace, par exemple simplement du fait de la respiration d'un sujet.

Un but de la présente invention est ainsi de pouvoir localiser un volume de cellules in-vivo, telles que des cellules cancéreuses, de manière dynamique spatialement et temporellement. De plus, l'invention devrait présenter une aptitude à piloter aussi dynamiquement une cible (virtuelle ou non) de faisceau d'une installation de positionnement d'un appareillage annexe sur une zone in-vivo, en vue par exemple du bon planning ou suivi d'une potentielle séance d'irradiation desdites cellules tout en épargnant précisément des tissus sains périphériques.

Enfin, un problème alternatif de l'invention concerne le fait que la visualisation et la localisation d'une tumeur par exemple doit être effectuée en préservant la santé mais aussi le confort et la quiétude du patient afin de lui éviter tout stress complémentaire à un potentiel diagnostic voire une thérapie plus lourde et astreignante. Ainsi, toute irradiation invasive ou du moins nuisible, en supplément à celle liée à une possible radiothérapie ou une thérapie similaire, ainsi que l'usage de méthodes de maintien du patient, de marquage ou autre contrainte infligée au patient doit être minimisé, et idéalement éliminé.

L'invention propose ainsi un dispositif approprié à résoudre cet ensemble de problèmes et décrit au travers de la revendication 1.

Ainsi, l'invention prévoit un dispositif de guidage d'ultrasons apte à être piloté pour visualiser et localiser spatialement une zone limitée, la dite zone étant une zone in-vivo d'un sujet, comprenant :
- une pluralité de parties émettrices/réceptrices d'ultrasons, chacune étant reliée à au moins une sonde à ultrasons,
- les parties émettrices/réceptrices d'ultrasons forment un réseau de canaux de guidage d'ultrasons vers le sujet, lesdits canaux étant distinctement espacés sur une surface d'un plan contre lequel au moins une partie du sujet est superficiellement appuyée,
- un module de contrôle est relié à une unité de commutation qui active le guidage d'ultrasons au travers d'au moins un des canaux dits activés et désactive le guidage d'ultrasons au travers des autres canaux dits désactivés.

En raison de l'intégration avantageuse d'une ou plusieurs sondes à ultrasons, il est ainsi possible de visualiser et localiser la zone limitée (volume de cellules tumorales par exemple) de façon continue et en temps réel, même chez un patient lourdement malade et affaibli, car les ultrasons n'ont pas d'effets nocifs et affaiblissants connus. Si le patient bouge par rapport à une table où il est allongé, le support mobile initialement disposé pour permettre d'inclure la zone visée et son voisinage dans son champ de vision (eux-mêmes en possible mouvement, par exemple par de simples raisons respiratoire, de flux sanguin ou d'autres aspects dynamiques in-vivo) assure un déplacement d'un champ de vision d'un canal au champ de vision d'un autre canal afin de toujours cadrer la zone visée dans le meilleur champ de vision possible. Aucune prédisposition de ou des sondes n'est ainsi nécessaire en début d'une phase de visualisation. Seul, un opérateur ou un algorithme de visualisation et de localisation permet d'engager simplement le guidage des ultrasons par un ou des canaux adaptés à couvrir la zone « tracée » dans un champ de vision approprié.

Il est à noter que le traçage spatial de cette zone par la sonde peut être mesurable métriquement dans un référentiel absolu afin par exemple de guider (de façon synchrone et également dynamique) un faisceau annexe radio-thérapeutique exactement sur la zone à irradier. En effet, les sondes à ultrasons actuelles permettent une vision bi- ou tridimensionnelle et donc, une fois que la zone limitée (tumeur) est localisée au travers d'un canal, il est possible de recalculer sa position par la simple connaissance de la position du canal activé par une sonde par rapport au plan d'appui du patient (fixe ou du moins spatialement connu dans le référentiel de l'installation annexe de diagnostic/thérapie). Ladite localisation de la zone ciblée et donc un pilotage possible d'une cible d'un faisceau d'un appareillage annexe devant être cadrée sur ladite zone ciblée est donc fort exacte spatialement et absolument dynamique temporellement.

Un ensemble de sous-revendications présente également des avantages de l'invention sous plusieurs aspects.

Des exemples de réalisation du dispositif selon l'invention sont fournis à l'aide des figures suivantes :
- Figure 1: premier mode de réalisation d'un dispositif de guidage d'ultrasons selon l'invention,
- Figure 2: second mode de réalisation d'un dispositif de guidage d'ultrasons selon l'invention,
- Figure 3: exemple de réseau bidimensionnel de canaux.

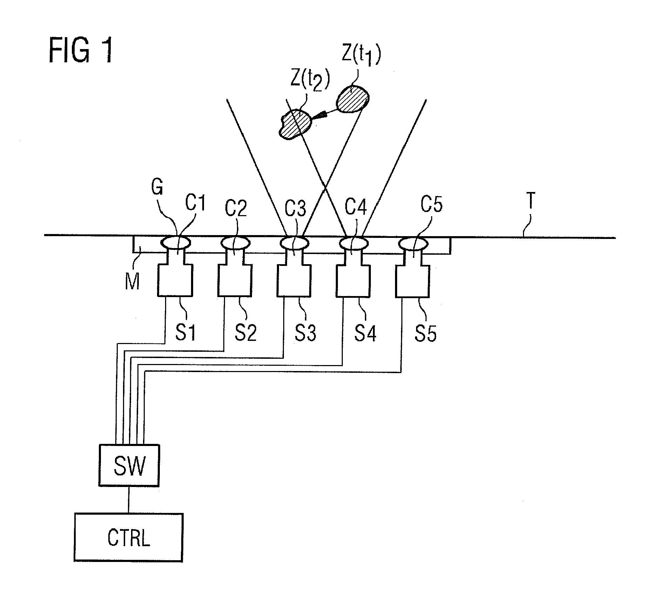

**Figure 1** présente un premier mode de réalisation d'un dispositif de guidage d'ultrasons étant apte à être piloté pour visualiser et localiser spatialement une zone limitée (Z), la dite zone étant une zone in-vivo d'un sujet et donc potentiellement mobile.

Ledit dispositif comprend :
- une pluralité de parties émettrices/réceptrices d'ultrasons, chacune étant reliée à au moins une sonde à ultrasons (S, S1, S2, S3,...),
- les parties émettrices/réceptrices d'ultrasons forment un réseau de canaux (C1, C2, C3, ...) de guidage d'ultrasons vers le sujet, lesdits canaux étant distinctement espacés sur une surface d'un plan (T) contre lequel au moins une partie du sujet est superficiellement appuyée,
- un module de contrôle (CTRL) est relié à une unité de commutation (SW) qui active le guidage d'ultrasons au travers d'au moins un des canaux dits activés et désactive le guidage d'ultrasons au travers des autres canaux dits désactivés.

A la figure 1, deux positions distinctes (Z(t1), Z(t2)) de la zone limitée (Z) sont représentées pour deux instants (t1, t2) entre lesquels ladite zone a subi un déplacement. Le dispositif selon l'invention comprend cinq sondes à ultrasons (S1, S2, S3, S4, S5) reliées chacune à un canal respectif (C1, C2, C3, C4, C5). A l'instant (t1), l'unité de commutation active le quatrième canal (C4) relié à la quatrième sonde à ultrasons (S4) car la zone (Z(t1)) se trouve au milieu de son champ de vision échographique. Les autres canaux ainsi restants (C1, C2, C3, C5) et leurs sondes respectives (S1, S2, S3, S5) sont alors désactivables, car leur champ de vision n'est pas cadré sur la zone aussi bien que le champ de vision du quatrième canal (C4). A l'instant (t2), après déplacement de la zone (Z), l'unité de commutation active ainsi le troisième canal (C3) relié à la troisième sonde à ultrasons (S3) car la zone (Z(t2)) se trouve au milieu de son champ de vision échographique. C'est à partir module de contrôle qu'arrivent des signaux de monitoring des canaux et sondes respectives et que le choix de commutation est ordonné.

Le dispositif peut prévoir que le module de contrôle (CTRL) est un séquenceur permettant un balayage périodique d'ultrasons par des activations séquentielles de canaux sur au moins une partie du réseau. De cette façon, il est possible de rapidement contrôler quel est le canal permettant le meilleure visualisation et localisation de la zone limitée (Z). Il est aussi possible d'organiser la séquence par groupe de canaux actifs afin d'accélérer le balayage ou bien afin de créer des champs de vision partiellement superposés pour éviter des zones d'ombre.

Le dispositif selon l'invention prévoit ainsi idéalement que le module de contrôle (CTRL) comprend une unité de reconnaissance et de localisation de la zone limitée (Z) qui impose une sélection d'un ou de canaux activables. A cet effet, l'unité de reconnaissance et de localisation est régie sous un mode opérationnel manuel commutable (essentiellement en cas d'initiation du guidage pour trouver une zone particulière que le module de contrôle devra reconnaître et tracer dynamiquement) sur un mode opérationnel automatique pour lequel un algorithme est exécuté par ladite unité. De cette façon, le guidage des ultrasons sur la zone mobile est rendu fort dynamique.

Le dispositif selon l'invention prévoit également que les canaux d'ultrasons présentent une impédance acoustique globale avoisinée de celle d'une embouchure (par bouchon, coussin, matelas (M), etc.) d'un gel acoustique (G) assurant un guide acoustique libre de tout interstice d'air à la surface d'appui du sujet sur une section de canal. Ceci permet de ne pas introduire d'artefact gênant dans un enregistrement échographique d'une des sondes.

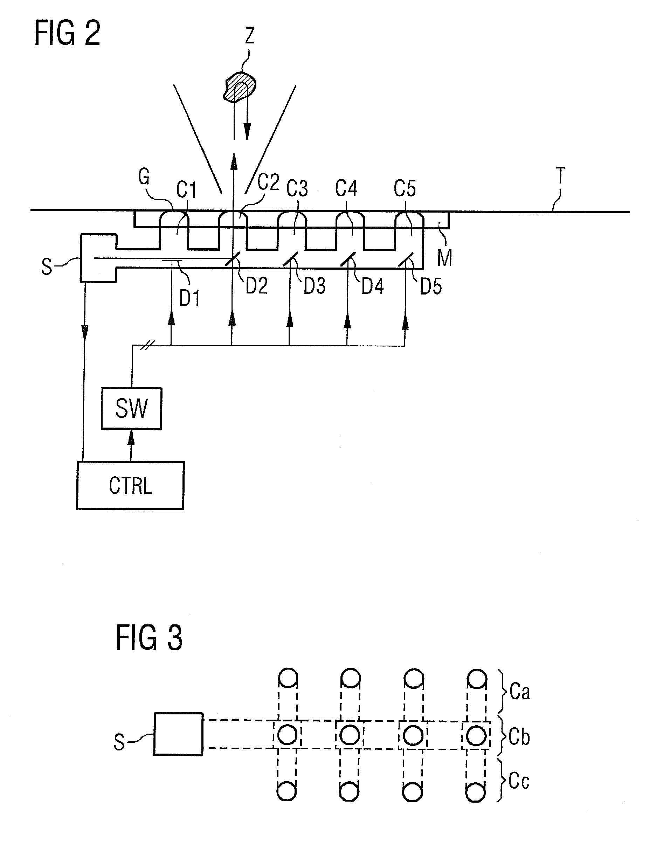

Ces dernières caractéristiques sont ainsi applicables au dispositif selon l'exemple de la figure 1, pour lequel chaque canal est relié à sa propre sonde à ultrasons. Elles sont aussi principalement applicables à la **figure 2** présentant un second mode de réalisation du dispositif sur la base de l'exemple de la figure 1 comprenant cinq canaux, pour lequel toutefois plusieurs canaux (C1, C2...) sont reliés à une unique sonde à ultrasons (S) et disposent chacun d'un moyen de commutation (D1, D2, ...) de guidage d'ultrasons. Le module de contrôle (CTRL) - et son unité de reconnaissance et de localisation - est ainsi relié à la sonde pour analyser et contrôler la commutation sur le canal le plus approprié à la visualisation et la localisation de la zone limitée (Z). Du fait de l'emploi d'une seule sonde à ultrasons, le dispositif est plus simple et moins coûteux. Les moyens de commutations activés par l'unité de commutation (SW) commandé par le module de contrôle (CTRL) peuvent être des guides d'ondes orientables, inclinables, obturables, etc. Ils ne feront cependant pas l'objet d'une plus grande description dans la présente invention.

Les dispositifs selon figures 1 et 2 présentent un réseau de canaux réparti monodimensionnellement suivant une direction rectiligne du plan (T). Ils pourraient aussi être disposés sur un axe à courbure afin de positionner les champs de vision des canaux ou sondes sur un espace plus convexe, tel un tronc d'un patient. En particulier et tel que représenté à la **figure 3** sur la base de la figure 2 à mono-sonde et multi-canaux, le réseau de canaux peut aussi être réparti bidimensionnellement suivant deux directions orthogonales du plan (T), et peut ainsi idéalement présenter une courbure selon au moins une des directions afin d'incliner des directions principales de canaux d'ultrasons en bord de réseau vers un volume couvrant une partie centrale du réseau. Dans le cas de la figure 3, trois rangées de canaux (Ca, Cb, Cc) comprenant chacune quatre canaux espacés linéairement peuvent ainsi être disposées à des hauteurs diverses ou/et des axes normaux aux sections de canaux de chaque rangée peuvent être disposés convergents vers un point ou une ligne de référence.

Dans le cadre de tous les modes de réalisation du dispositif selon l'invention, une utilisation fort avantageuse dudit dispositif comme moyen de localisation de la zone limitée in-vivo est rendue possible, pour laquelle le plan est une table sur laquelle, en tant que sujet, un être vivant est allongé et la zone limitée est un ensemble de cellules in-vivo à localiser spatialement au moyen de coordonnées spatiales instantanément délivrées par commutation et activation de canaux d'ultrasons et de moyen de calcul de la position instantanée de la sonde par rapport à un repère tridimensionnel lié au plan. A cet effet, tout canal dit activé est couplé à l'unité de visualisation ou/et un traçage spatial de l'ensemble des cellules in-vivo, la dite unité étant pilotée par un opérateur ou un algorithme de reconnaissance. Avantageusement, l'unité de visualisation ou/et un traçage spatial est reliée à une unité de commande de positionnement d'une installation de type radiothérapeutique dont la cible est positionnable sur la zone limitée in-vivo, et ce de manière dynamique et précise. Il est à prévoir dans de dernier cas que le dispositif ou ses éléments à base électronique (sonde) ne soit pas disposés dans le champ d'irradiation du faisceau annexe de ciblage. Le mode de réalisation selon la figure 2 présentant un éloignement de la sonde à ultrasons hors de la zone potentielle d'irradiation s'y prête ainsi parfaitement.

## Revendications

1. Dispositif de guidage d'ultrasons apte à être piloté pour visualiser et localiser spatialement une zone limitée (Z), la dite zone étant une zone in-vivo d'un sujet, comprenant :
- une pluralité de parties émettrices/réceptrices d'ultrasons, chacune étant reliée à au moins une sonde à ultrasons (S, S1, S2, S3,...),
- les parties émettrices/réceptrices d'ultrasons forment un réseau de canaux (C1, C2, C3, ...) de guidage d'ultrasons vers le sujet, lesdits canaux étant distinctement espacés sur une surface d'un plan (T) contre lequel au moins une partie du sujet est superficiellement appuyée,
- un module de contrôle (CTRL) est relié à une unité de commutation (SW) qui active le guidage d'ultrasons au travers d'au moins un des canaux dits activés et désactive le guidage d'ultrasons au travers des autres canaux dits désactivés.

2. Dispositif selon revendication 1, pour lequel le module de contrôle (CTRL) est un séquenceur permettant un balayage périodique d'ultrasons par des activations séquentielles de canaux sur au moins une partie du réseau.

3. Dispositif selon revendication 1 ou 2, pour lequel le module de contrôle (CTRL) comprend une unité de reconnaissance et de localisation de la zone limitée (Z) qui impose une sélection de canaux activables.

4. Dispositif selon revendication 3, pour lequel l'unité de reconnaissance et de localisation est régie sous un mode opérationnel manuel commutable sur un mode opérationnel automatique pour lequel un algorithme est exécuté par ladite unité.

5. Dispositif selon une des revendications précédentes, pour lequel les canaux d'ultrasons présentent une impédance acoustique globale avoisinée de celle d'une embouchure de gel acoustique (G) assurant un guide acoustique libre de tout interstice d'air à la surface d'appui du sujet sur une section de canal.

6. Dispositif selon une des revendications précédentes, pour lequel chaque canal est relié à une sonde à ultrasons.

7. Dispositif selon une des revendications précédentes 1-5, pour lequel plusieurs canaux sont reliés à une sonde à ultrasons et disposent chacun d'un moyen de commutation de guidage d'ultrasons.

8. Dispositif selon une des revendications précédentes, pour lequel le réseau de canaux est réparti monodimensionnellement suivant une direction du plan (T).

9. Dispositif selon une des revendications précédentes, pour lequel le réseau de canaux est réparti bidimensionnellement suivant deux directions du plan (T), et idéalement présente une courbure selon au moins une direction afin d'incliner des directions principales de canaux en bord de réseau vers un volume couvrant une partie centrale du réseau.

10. Utilisation du dispositif selon une des revendications précédentes comme moyen de localisation de la zone limitée in-vivo, pour laquelle le plan est une table sur laquelle, en tant que sujet, un être vivant est allongé et la zone limitée est un ensemble de cellules in-vivo à localiser spatialement au moyen de coordonnées spatiales instantanément délivrées par commutation et activation de canaux d'ultrasons et de moyen de calcul de la position instantanée de la sonde par rapport à un repère tridimensionnel lié au plan.

11. Utilisation selon revendication 10, pour laquelle un canal dit activé est couplé à une unité de visualisation ou/et un traçage spatial de l'ensemble des cellules in-vivo, la dite unité étant pilotée par un opérateur ou un algorithme de reconnaissance.

12. Utilisation selon revendication 9 ou 10, pour laquelle l'unité de visualisation ou/et un traçage spatial est reliée à une unité de commande de positionnement d'une installation de type radiothérapeutique dont la cible est positionnable sur la zone limitée in-vivo.
